(19) Europäisches Patentamt
European Patent Office
Office européen des brevets

(11) **EP 4 052 587 A1**

(12) **EUROPEAN PATENT APPLICATION**
published in accordance with Art. 153(4) EPC

(43) Date of publication:
**07.09.2022 Bulletin 2022/36**

(21) Application number: **20882088.6**

(22) Date of filing: **30.10.2020**

(51) International Patent Classification (IPC):
**A23J 3/16** $^{(2006.01)}$     **C12N 9/16** $^{(2006.01)}$

(52) Cooperative Patent Classification (CPC):
**A23J 3/16; C12N 9/16**

(86) International application number:
**PCT/KR2020/015092**

(87) International publication number:
**WO 2021/086138 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.10.2019 KR 20190136872**

(71) Applicant: **CJ Cheiljedang Corporation
Seoul 04560 (KR)**

(72) Inventors:
• **SEO, Hyojeong**
  **Seoul 04560 (KR)**
• **LEE, In**
  **Seoul 04560 (KR)**
• **HAN, Sungwook**
  **Seoul 04560 (KR)**
• **KIM, Seong Bo**
  **Seoul 04560 (KR)**

(74) Representative: **Grünecker Patent- und
Rechtsanwälte
PartG mbB
Leopoldstraße 4
80802 München (DE)**

(54) **COMPOSITION FOR PREPARING SOY PROTEIN CONCENTRATE HAVING REDUCED PHYTIC ACID, AND USE THEREOF**

(57) The present invention relates to a composition for preparing a soy protein concentrate having reduced phytic acid, and a use thereof. According to the use of a composition for preparing a soy protein concentrate having reduced phytic acid, according to one embodiment, or to a preparation method according to one embodiment, a soy protein concentrate having reduced phytic acid can be efficiently prepared, and thus the amount of phosphorus that can be utilized by animals can be increased, the content ratio of low-molecular-weight peptides increases, and soy protein concentrate digestibility in animals can be improved, and a feed composition containing a soy protein concentrate having reduced phytic acid, according to one embodiment, can have excellent digestibility.

EP 4 052 587 A1

**Description**

[TECHNICAL FIELD]

The present invention relates to a composition for preparing a soy protein concentrate with reduced phytic acid and uses thereof.

[BACKGROUND ART]

Soy (soybean) protein is a low-cost vegetable protein, and it is converted into various forms other than edible soybean itself, so that soy protein products such as soy flour, defatted soy meal, a soy protein concentrate, a soy protein isolate, texturized soy protein, and the like are used in various food processing and manufacturing processes. These soy protein products are used as additives in various processed products such as meat, grains, confectionery, beverages, baby food, dairy products and so-called second-generation soy food for excellent nutritional, functional and economical reasons and they are prepared as various secondary processed products.

However, soybean protein usually contains phytic acid, and phytic acid is known to combine with nutritionally important trace metals such as calcium, magnesium, iron and zinc to form poorly soluble compounds, thereby having inhibitory action on digestive enzymes such as $\alpha$-amylase, trypsin or pepsin, so its removal from food is desirable.

Methods for removing phytic acid known to date include ultrafiltration, ion exchange resin method and chemical method, and the like. In the ultrafiltration, there is a problem that washing must be repeated regularly, since peptides accumulate on the membrane surface during operation, which slows the filtration rate, and phytic acid includes phosphoric acid residues, so it may be removed by treating the protein solution with an anion exchange resin using the ion exchange resin method, but the adsorption of soybean protein to the protein resin is also remarkable, so the ion exchange resin method results in a complicated operation and a decrease in the amount of protein. As a chemical method, it is possible to extract and remove soybean protein by treating it with a strong acid such as hydrochloric acid or trichloroacetic acid or a strong base such as sodium hydroxide, but also in this method, manufacturing operation is complicated, and it requires effort to dispose of a large amount of waste liquid, and manufacturing cost is increased, and protein is denatured by acid-base treatment, and it negatively affects protein digestion.

Accordingly, there is a need to develop a composition that can be used to efficiently remove phytic acid from a soy protein concentrate and a method for preparation of a soy protein concentration from which phytic acid is removed, and a feed composition that can replace fish meal using a soy protein concentrate.

[Prior art]

(Patent document 1) U.S. Patent Publication No. 2010-0279367

[DISCLOSURE]

[TECHNICAL PROBLEM]

An object of the present application is to provide a composition for preparing a soy protein concentrate with reduced phytic acid.

Another object of the present application is to provide a method for preparation of a soy protein concentrate with reduced phytic acid.

Other object of the present application is to provide a soy protein concentrate with reduced phytic acid prepared by reacting the composition for preparing a soy protein concentrate with reduced phytic acid.

Other object of the present application is to provide a feed composition comprising a soy protein concentrate with reduced phytic acid.

[TECHNICAL SOLUTION]

In the present description, "soy protein concentrate (SPC)" means one which a soybean-derived protein content is concentrated by removing soluble non-protein substances from defatted soybeans, and it may be utilized as a high-protein feed raw material with a high protein content using soybean meal which is a by-product remaining after making cooking oil with beans as a raw material. Soy protein concentrate is classified into a representative vegetable high-protein material with fermented soybean meal, and it can be an alternative to fish meal, which has been used as a major protein source. For example, soy protein concentrate may be prepared by eluting defatted soybeans with water close to the isoelectric point (pH 4 to 5) or 20 to 80% ethanol, removing water-soluble and non-protein substances such as carbohydrates and concentrating mainly globulin-like protein, and the soy protein concentrate may be a soybean protein inclusion comprising protein of 50 to 90 % by weight based on the dried matter in which moisture is removed.

In the present description, "about" or "approximately" may be generally understood as meaning including a value or range within 20%, within 10%, within 5%, within 4%, within 3%, within 2%, within 1%, or within 0.5%, above and below a given value or range.

In the present description, "protein solubility" refers to a degree that protein is dissolved in water, and the more hydrophilic amino acids are present on the surface, the more they interact with the ionic groups of the solvent to increase the protein solubility.

In the present description, "soy protein concentrate with reduced phytic acid" means that the phytic acid content is reduced than the soy protein concentrate raw material (or, soy protein concentrate raw material not comprising phytase).

The soy protein concentrate with reduced phytic acid according to one example may have a phytic acid content reduced by about 35% or more, 40% or more, 50% or more, 60% or more, 70% or more, 75% or more or 80% or more, 0.1 to 100%, 1 to 100%, 10 to 100%, 20 to 100%, 25 to 100%, 30 to 100%, 35 to 100%, 40 to 100%, 50 to 100%, 60 to 100%, 65 to 100%, 70 to 100%, 0.1 to 90%, 1 to 90%, 10 to 90%, 20 to 90%, 25 to 90%, 30 to 90%, 35 to 90%, 40 to 90%, 50 to 90%, 60 to 90%, 65 to 90%, 70 to 90%, 0.1 to 85%, 1 to 85%, 10 to 85%, 20 to 85%, 25 to 85%, 30 to 85%, 35 to 85%, 40 to 85%, 50 to 85%, 60 to 85%, 65 to 85%, 70 to 85%, 0.1 to 80%, 1 to 80%, 10 to 80%, 20 to 80%, 25 to 80%, 30 to 80%, 35 to 80%, 40 to 80%, 50 to 80%, 60 to 80%, 65 to 80%, 70 to 80%, 0.1 to 75%, 1 to 75%, 10 to 75%, 20 to 75%, 25 to 75%, 30 to 75%, 35 to 75%, 40 to 75%, 50 to 75%, 60 to 75%, 65 to 75%, or 70 to 75% based on the soy protein concentrate raw material.

Phytic acid is IP6 (myo-inositol hexaphosphate) and consists of a myo-inositol ring and 6 phosphate groups, and these phosphate groups are attached symmetrically. It is present in plants and in particular, it is contained in beans and plants. Phytic acid forms a complex with protein, reducing the nutritional value of soybean protein, reducing the action of polyvalent metal ions such as calcium, iron, and zinc, and preventing absorption into the body, and pepsin or trypsin interferes with digestive enzyme activity in the stomach, so the soy protein concentrate with reduced phytic acid according to one example has a low phytic acid content and therefore, it may be usefully used as a nutritionally excellent food additive.

Otherwise, the present invention will be described in more detail.

One aspect may provide a composition for preparing a soy protein concentrate with reduced phytic acid (or a composition for reducing phytic acid of a soy protein concentrate), comprising a soy protein concentrate and phytase.

In the present description, 'composition for preparing a soy protein concentrate with reduced phytic acid' may mean the same one as 'composition for reducing (decreasing) phytic acid of a soy protein concentrate'.

The composition for preparing a soy protein concentrate with reduced phytic acid according to one example comprises phytase, and the content of the phytase may be 0.001 to 10% by weight, 0.005 to 10% by weight, 0.01 to 10% by weight, 0.025 to 10% by weight, 0.03 to 10% by weight, 0.05 to 10% by weight, 0.075 to 10% by weight, 0.1 to 10% by weight, 0.25 to 10% by weight, 0.3 to 10% by weight, 0.5 to 10% by weight, 0.001 to 5% by weight, 0.005 to 5% by weight, 0.01 to 5% by weight, 0.025 to 5% by weight, 0.03 to 5% by weight, 0.05 to 5% by weight, 0.075 to 5% by weight, 0.1 to 5% by weight, 0.25 to 5% by weight, 0.3 to 5% by weight, 0.5 to 5% by weight, 0.001 to 1% by weight, 0.005 to 1% by weight, 0.01 to 1% by weight, 0.025 to 1% by weight, 0.03 to 1% by weight, 0.05 to 1% by weight, 0.075 to 1% by weight, 0.1 to 1% by weight, 0.25 to 1% by weight, 0.3 to 1% by weight, 0.5 to 1% by weight, 0.001 to 0.5% by weight, 0.005 to 0.5% by weight, 0.01 to 0.5% by weight, 0.025 to 0.5% by weight, 0.03 to 0.5% by weight, 0.05 to 0.5% by weight, 0.075 to 0.5% by weight, 0.1 to 0.5% by weight, 0.25 to 0.5% by weight, 0.3 to 0.5% by weight, 0.5 to 0.5% by weight, 0.001 to 0.3% by weight, 0.005 to 0.3% by weight, 0.01 to 0.3% by weight, 0.025 to 0.3% by weight, 0.03 to 0.3% by weight, 0.05 to 0.3% by weight, 0.075 to 0.3% by weight, 0.1 to 0.3% by weight, 0.25 to 0.3% by weight, 0.001 to 0.25% by weight, 0.005 to 0.25% by weight, 0.01 to 0.25% by weight, 0.025 to 0.25% by weight, 0.03 to 0.25% by weight, 0.05 to 0.25% by weight, 0.075 to 0.25% by weight, or 0.1 to 0.25% by weight, based on the weight of the soy protein concentrate (for example, soy protein concentrate raw material).

The phytase is one kind of phosphatase producing myo-inositol and inorganic phosphate by hydrolyzing phytic acid, and the composition comprising phytase, according to one example, may effectively reduce the phytic acid content of the soy protein concentrate.

In case of the composition comprising phytase in a content within the above range, during preparation of the soy protein concentrate with reduced phytic acid, the phytic acid content based on the amount of phytase added may be effectively reduced, and the composition comprising phytase in the above range may prepare a soy protein concentrate with reduced phytic acid economically.

The composition for preparing a soy protein concentrate with reduced phytic acid comprising phytase in the above range may excellently (significantly) induce (1) reduction of the phytic acid content; (2) increase of free phosphorus content; and/or (3) digestibility increase of the soy protein concentrate, than a composition comprising phytase outside the above range.

In one example, the moisture content of the composition for preparing a soy protein concentrate with reduced phytic acid may be 20 % by weight or more, 30 % by weight or more, 40 % by weight or more, 45 % by weight or more, 50 % by weight or more, 55 % by weight or more, 95 % by weight or less, 90 % by weight or less, 85 % by weight or less, 80 % by weight or less, 75 % by weight or less, 70 % by weight or less, 65 % by weight or less, 60 % by weight or less, 30 to 95% by weight, 35 to 95% by weight, 40 to 95% by weight, 45 to 95% by weight, 50 to 95% by weight, 55 to 95% by weight, 30 to 90% by weight, 35 to 90% by weight, 40 to 90% by weight, 45 to 90% by weight, 50 to 90% by weight, 55 to 90% by weight, 30 to 85% by weight, 35 to 85% by weight, 40 to 85% by weight, 45 to 85% by weight, 50 to 85% by weight, 55 to 85% by weight, 30 to 80% by weight, 35 to 80% by weight, 40 to 80% by weight, 45 to 80% by weight, 50 to 80% by weight, 55 to 80% by weight, 30 to 75% by weight, 35 to 75% by weight, 40 to 75% by weight, 45 to 75% by weight, 50 to 75% by weight, 55 to 75% by weight, 30 to 70% by weight, 35 to 70% by weight, 40 to 70% by weight, 45 to 70% by weight, 50 to 70% by weight, 55 to 70% by weight, 30 to 65% by weight, 35 to 65% by weight, 40 to 65% by weight, 45 to 65% by weight, 50 to 65% by weight, 55 to 65% by weight, 30 to 60% by weight, 35 to 60% by weight, 40 to 60% by weight, 45 to 60% by weight, 50 to 60% by weight, 55 to 60% by weight, 30 to 55% by weight, 35 to 55% by weight, 40 to 55% by weight, 45 to 55% by weight, or 50 to 55% by weight, based on the weight of the total composition.

The composition for preparing a soy protein concentrate with reduced phytic acid having the moisture content in the above range may excellently (significantly) induce (1) reduction of the phytic acid content; (2) increase of free phosphorus content; and/or (3) digestibility increase of the soy protein concentrate, than a composition comprising the moisture content outside the above range.

In one example, the pH of the composition for preparing a soy protein concentrate with reduced phytic acid may be 7 or less, 6.8 or less, 6.5 or less, 6 or less, 5.5 or less, 5 or less, 4.75 or less, 4.5 or less, 4 or less, 1 or more, 1.5 or more, 2 or more, 2.5 or more, 3 or more, 3.5 or more, 4 or more, 4.5 or more, pH 2 to 7, pH 2.5 to 7, pH 3 to 7, pH 3.5 to 7, pH 4 to 7, pH 4.25 to 7, pH 4.5 to 7, pH 2 to 6.8, pH 2.5 to 6.8, pH 3 to 6.8, pH 3.5 to 6.8, pH 4 to 6.8, pH 4.25 to 6.8, pH 4.5 to 6.8, pH 2 to 6.5, pH 2.5 to 6.5, pH 3 to 6.5, pH 3.5 to 6.5, pH 4 to 6.5, pH 4.25 to 6.5, pH 4.5 to 6.5, pH 2 to 6, pH 2.5 to 6, pH 3 to 6, pH 3.5 to 6, pH 4 to 6, pH 4.25 to 6, pH 4.5 to 6, pH 2 to 5.5, pH 2.5 to 5.5, pH 3 to 5.5, pH 3.5 to 5.5, pH 4 to 5.5, pH 4.25 to 5.5, pH 4.5 to 5.5, pH 2 to 5, pH 2.5 to 5, pH 3 to 5, pH 3.5 to 5, pH 4 to 5, pH 4.25 to 5, pH 4.5 to 5, pH 2 to 4.75, pH 2.5 to 4.75, pH 3 to 4.75, pH 3.5 to 4.75, pH 4 to 4.75, pH 4.25 to 4.75, pH 4.5 to 4.75, pH 2 to 4.5, pH 2.5 to 4.5, pH 3 to 4.5, pH 3.5 to 4.5, pH 4 to 4.5, or pH 4.25 to 4.5.

The composition for preparing a soy protein concentrate with reduced phytic acid having the pH in the above range may excellently (significantly) induce (1) reduction of the phytic acid content; (2) increase of free phosphorus content; and/or (3) digestibility increase of the soy protein concentrate, than a composition comprising the pH outside the above range.

According to one example, a soy protein concentrate with reduced phytic acid may be prepared by reacting and drying the composition for preparing a soy protein concentrate with reduced phytic acid. The composition for preparing a soy protein concentrate with reduced phytic acid according to one example may effectively reduce phytic acid and increase the ratio of low-protein peptide to increase the digestibility of animals, and it is possible to prepare a soy protein concentrate with easy quality control, and this may be added to the feed to increase the digestibility of animals and increase the phosphorus content without additional phosphorus addition.

Another aspect provides a method for preparation of a soybean protein concentrate with reduced phytic acid, comprising reacting the soybean protein concentrate with reduced phytic acid.

The reacting the composition for preparing a soy protein concentrate with reduced phytic acid may be incubating (or reacting) the composition at a temperature of 20 to 80°C, 25 to 80°C, 30 to 80°C, 40 to 80°C, 50 to 80°C, 55 to 80 °C, 20 to 70°C, 25 to 70°C, 30 to 70°C, 40 to 70°C, 50 to 70°C, 55 to 70°C, 20 to 65°C , 25 to 65°C, 30 to 65°C, 40 to 65°C, 50 to 65°C, 55 to 65°C, 20 to 60°C, 25 to 60°C, 30 to 60°C, 40 to 60°C, 50 to 60°C, or 55 to 60°C, for 0.5 to 24 hours, 1 to 24 hours, 2 to 24 hours, 3 to 24 hours, 4 to 24 hours, 0.5 to 18 hours, 1 to 18 hours, 2 to 18 hours, 3 to 18 hours, 4 to 18 hours, 0.5 to 12 hours, 1 to 12 hours, 2 to 12 hours, 3 to 12 hours, 4 to 12 hours, 0.5 to 8 hours, 1 to 8 hours, 2 to 8 hours, 3 to 8 hours, 4 to 8 hours, 0.5 to 6 hours, 1 to 6 hours, 2 to 6 hours, 3 to 6 hours, or 4 to 6 hours.

When the enzymatic reaction is performed under the condition within the above range, without protein denaturation of the soy protein concentrate, a soy protein concentrate with reduced phytic acid may be effectively prepared.

The method for preparation may further comprise drying the reactant reacted in the above step. The drying may be performed by a method of natural air drying, hot air drying, vacuum drying, spray drying and/or freeze drying. The enzymatic reaction of phytase may be terminated by the drying process.

The method for preparation may further comprise pulverizing the dried soy protein concentrate after the drying, and the pulverizing may be pulverizing into various sizes according to the purpose of using the soy protein concentrate, and for example, it may be pulverizing using a hammer mill.

Other aspect may provide a method for preparation of a soy protein concentrate with reduced phytic acid, comprising the following steps:

adjusting the pH of the soy protein concentrate to 6.8 or less by adding acid into the soy protein concentrate; and adjusting the final moisture content of the soy protein concentrate to 40 % by weight or more by adding phytase and water.

Other aspect may provide a method for preparation of a soy protein concentrate with reduced phytic acid, comprising adjusting the moisture content of the soy protein concentrate to 40 % by weight or more and adjusting the pH of the soy protein concentrate to 6.8 or less, by adding a mixture comprising phytase, water and/or acid to the soy protein concentrate.

Other aspect may provide a method for preparation of a soy protein concentrate with reduced phytic acid, comprising adjusting the pH of the soy protein concentrate to 6.8 or less by adding a mixture comprising phytase, water and/or acid to the soy protein concentrate, and adjusting the final moisture content of the soy protein concentrate to 40 % by weight or more by additionally adding phytase and/or water.

According to one example, the soy protein concentrate, which is a raw material before adjusting the moisture content and/or pH may be used by continuously receiving the same type of soybean from the same region to maintain the same quality. For example, preparation of the soybean protein concentrate may be performed by washing soybean protein with alcohol and extracting bean oil using an organic solvent such as hexane, and the like, and then further adding alcohol to the remaining defatted soybean meal to remove non-protein components such as water-soluble and non-water-soluble carbohydrates other than protein.

According to one example, the adjusting the moisture content may be performed by adding phytase and water to the soy protein concentrate or performed by adding water to the soy protein concentrate. The adjusting the moisture content may be adjusting the moisture content of the soy protein concentrate to 20 % by weight or more, 30 % by weight or more, 40 % by weight or more, 45 % by weight or more, 50 % by weight or more, 55 % by weight or more, 95 % by weight or less, 90 % by weight or less, 85 % by weight or less, 80 % by weight or less, 75 % by weight or less, 70 % by weight or less, 65 % by weight or less, 60 % by weight or less, 30 to 95% by weight, 35 to 95% by weight, 40 to 95% by weight, 45 to 95% by weight, 50 to 95% by weight, 55 to 95% by weight, 30 to 90% by weight, 35 to 90% by weight, 40 to 90% by weight, 45 to 90% by weight, 50 to 90% by weight, 55 to 90% by weight, 30 to 85% by weight, 35 to 85% by weight, 40 to 85% by weight, 45 to 85% by weight, 50 to 85% by weight, 55 to 85% by weight, 30 to 80% by weight, 35 to 80% by weight, 40 to 80% by weight, 45 to 80% by weight, 50 to 80% by weight, 55 to 80% by weight, 30 to 75% by weight, 35 to 75% by weight, 40 to 75% by weight, 45 to 75% by weight, 50 to 75% by weight, 55 to 75% by weight, 30 to 70% by weight, 35 to 70% by weight, 40 to 70% by weight, 45 to 70% by weight, 50 to 70% by weight, 55 to 70% by weight, 30 to 65% by weight, 35 to 65% by weight, 40 to 65% by weight, 45 to 65% by weight, 50 to 65% by weight, 55 to 65% by weight, 30 to 60% by weight, 35 to 60% by weight, 40 to 60% by weight, 45 to 60% by weight, 50 to 60% by weight, 55 to 60% by weight, 30 to 55% by weight, 35 to 55% by weight, 40 to 55% by weight, 45 to 55% by weight, or 50 to 55% by weight, based on the weight of the total composition (for example, total weight of the soy protein concentrate comprising phytase, water and/or acid added).

According to one example, in the adjusting the moisture content of the soy protein concentrate, the moisture content may be measured by a commonly available method, and for example, it may be performed through a drying method (for example, atmospheric pressure drying method, and/or high temperature heating drying method, etc.), a distillation method, an electric moisture meter method, a near-infrared spectral absorption method, a gas chromatography method, a nuclear magnetic resonance absorption method, a capacity titration method, and/or a coulometric titration method. The moisture content of the soy protein concentrate (or soy protein concentrate raw material) before the moisture content is adjusted may be 5 to 30 % by weight, 5 to 25 % by weight, 5 to 20 % by weight, 5 to 15 % by weight, 5 to 12 % by weight, 5 to 10 % by weight, 6 to 9 % by weight, 7 to 8 % by weight, 15 to 25 % by weight, 20 to 25 % by weight, or 20 to 30 % by weight.

According to one example, in the adjusting the pH of the soy protein concentrate, the pH may be adjusted to 7 or less, 6.8 or less, 6.5 or less, 6 or less, 5.5 or less, 5 or less, 4.75 or less, 4.5 or less, 4 or less, 1 or more, 1.5 or more, 2 or more, 2.5 or more, 3 or more, 3.5 or more, 4 or more, 4.5 or more, pH 2 to 7, pH 2.5 to 7, pH 3 to 7, pH 3.5 to 7, pH 4 to 7, pH 4.25 to 7, pH 4.5 to 7, pH 2 to 6.8, pH 2.5 to 6.8, pH 3 to 6.8, pH 3.5 to 6.8, pH 4 to 6.8, pH 4.25 to 6.8, pH 4.5 to 6.8, pH 2 to 6.5, pH 2.5 to 6.5, pH 3 to 6.5, pH 3.5 to 6.5, pH 4 to 6.5, pH 4.25 to 6.5, pH 4.5 to 6.5, pH 2 to 6, pH 2.5 to 6, pH 3 to 6, pH 3.5 to 6, pH 4 to 6, pH 4.25 to 6, pH 4.5 to 6, pH 2 to 5.5, pH 2.5 to 5.5, pH 3 to 5.5, pH 3.5 to 5.5, pH 4 to 5.5, pH 4.25 to 5.5, pH 4.5 to 5.5, pH 2 to 5, pH 2.5 to 5, pH 3 to 5, pH 3.5 to 5, pH 4 to 5, pH 4.25 to 5, pH 4.5 to 5, pH 2 to 4.75, pH 2.5 to 4.75, pH 3 to 4.75, pH 3.5 to 4.75, pH 4 to 4.75, pH 4.25 to 4.75, pH 4.5 to 4.75, pH 2 to 4.5 pH, 2.5 to 4.5, pH 3 to 4.5, pH 3.5 to 4.5, pH 4 to 4.5, or pH 4.25 to 4.5.

In one example, the acid to be added in the adjusting the pH of the soy protein concentrate may be chloric acid, sulfuric acid, nitric acid and/or acetic acid, and it may be in a form of an aqueous solution.

According to one specific example, 1N HCl 40ml (or 100% HCl 1.458g) to adjust the pH of the soy protein concentrate raw material of 100g to 4.5, 1N HCl 33.5ml (or 100% HCl 1.221g) to adjust the pH to 4.75, 28ml (or 100% HCl 1.021g) to adjust the pH to 5, and 1N HCl 15ml (or 100% HCl 0.547g) to adjust the pH to 5.5 may be added, and the type and amount of the acid to be added may be changed according to the pH of the raw material.

According to one example, the pH of the soy protein concentrate before the pH is adjusted (or raw material of the soy protein concentrate) may be 5 to 8, 6 to 7.5, 6.5 to 7, or 6.5 to 6.8.

According to one example, the method for preparation may further comprise reacting (incubating), drying, and/or pulverizing the soy protein concentrate in which the moisture content and pH are adjusted. The reacting, drying and/or pulverizing are as described above.

According to one example, the "soy protein concentrate in which the moisture content and pH are adjusted" may be same as the "composition for preparing a soy protein concentrate with reduced phytic acid".

According to one example, the soy protein concentrate with reduced phytic acid may comprise phytic acid of less than 2 % by weight, less than 1.9 % by weight, less than 1.8 % by weight, less than 1.7 % by weight, less than 1.6 % by weight, less than 1.5 % by weight, less than 1.4 % by weight, less than 1.3 % by weight, less than 1.2 % by weight, less than 1.1 % by weight, less than 1 % by weight, less than 0.7 % by weight, less than 0.6 % by weight, less than 0.5 % by weight, less than 0.4 % by weight, less than 0.3 % by weight, less than 0.2 % by weight, 2 % by weight or less, 1.9 % by weight or less, 1.8 % by weight or less, 1.7 % by weight or less, 1.6 % by weight or less, 1.5 % by weight or less, 1.4 % by weight or less, 1.3 % by weight or less, 1.2 % by weight or less, 1.1 % by weight or less, 1 % by weight or less, 0.7 % by weight or less, 0.6 % by weight or less, 0.5 % by weight or less, 0.4 % by weight or less, 0.3 % by weight or less, 0.2 % by weight or less, 0.01 to 2% by weight, 0.05 to 2% by weight, 0.1 to 2% by weight, 0.2 to 2% by weight, 0.3 to 2% by weight, 0.01 to 1.5% by weight, 0.05 to 1.5% by weight, 0.1 to 1.5% by weight, 0.2 to 1.5% by weight, 0.3 to 1.5% by weight, 0.01 to 1% by weight, 0.05 to 1% by weight, 0.1 to 1% by weight, 0.2 to 1% by weight, 0.3 to 1% by weight, 0.01 to 0.8% by weight, 0.05 to 0.8% by weight, 0.1 to 0.8% by weight, 0.2 to 0.8% by weight, 0.3 to 0.8% by weight, 0.01 to 0.7% by weight, 0.05 to 0.7% by weight, 0.1 to 0.7% by weight, 0.2 to 0.7% by weight, 0.3 to 0.7% by weight, 0.01 to 0.6% by weight, 0.05 to 0.6% by weight, 0.1 to 0.6% by weight, 0.2 to 0.6% by weight, 0.3 to 0.6% by weight, 0.01 to 0.5% by weight, 0.05 to 0.5% by weight, 0.1 to 0.5% by weight, 0.2 to 0.5% by weight, 0.3 to 0.5% by weight, 0.01 to 0.4% by weight, 0.05 to 0.4% by weight, 0.1 to 0.4% by weight, 0.2 to 0.4% by weight, 0.3 to 0.4% by weight, 0.01 to 1.6 % by weight, 0.1 to 1.5 % by weight, 0.1 to 1.4 % by weight, 0.1 to 1 % by weight, 0.1 to 0.5 % by weight, 0.1 to 0.4 % by weight, 0.1 to 0.3 % by weight, 0.2 to 1.4 % by weight, 0.3 to 1.4 % by weight, 0.2 to 1.2 % by weight, 0.2 to 1 % by weight, 0.2 to 0.8 % by weight, 0.2 to 0.6 % by weight, 0.2 to 0.5 % by weight, 0.2 to 0.4 % by weight, 0.5 to 0.5 % by weight, 0.3 to 0.4 % by weight, 0.4 to 2% by weight, 0.4 to 1.5% by weight, 0.4 to 1% by weight, 0.4 to 0.8% by weight, 0.4 to 0.7% by weight, 0.4 to 0.6% by weight, or 0.4 to 0.5% by weight. The content of the phytic acid comprised in the soy protein concentrate with reduced phytic acid may be based on the total weight of the soy protein concentrate with reduced phytic acid (or based on the powder weight of the soy protein concentrate with reduced phytic acid in the dried form).

According to one example, the soy protein concentrate with reduced phytic acid may comprise free phosphorus of 0.1 to 2% by weight, 0.2 to 2% by weight, 0.3 to 2% by weight, 0.4 to 2% by weight, 0.5 to 2% by weight, 0.4 to 2% by weight, 0.1 to 1.5% by weight, 0.2 to 1.5% by weight, 0.3 to 1.5% by weight, 0.4 to 1.5% by weight, 0.5 to 1.5% by weight, 0.4 to 1.5% by weight, 0.1 to 1% by weight, 0.2 to 1% by weight, 0.3 to 1% by weight, 0.4 to 1% by weight, 0.5 to 1% by weight, 0.4 to 1% by weight, 0.1 to 0.8% by weight, 0.2 to 0.8% by weight, 0.3 to 0.8% by weight, 0.4 to 0.8% by weight, 0.5 to 0.8% by weight, 0.4 to 0.8% by weight, 0.1 to 0.7% by weight, 0.2 to 0.7% by weight, 0.3 to 0.7% by weight, 0.4 to 0.7% by weight, 0.5 to 0.7% by weight, 0.4 to 0.7% by weight, 0.1 to 0.6% by weight, 0.2 to 0.6% by weight, 0.3 to 0.6% by weight, 0.4 to 0.6% by weight, 0.5 to 0.6% by weight, or 0.4 to 0.6% by weight. The content of the free phosphorus comprised in the soy protein concentrate with reduced phytic acid may be based on the total weight of the soy protein concentrate with reduced phytic acid (or based on the powder weight of the soy protein concentrate with reduced phytic acid in the dried form).

According to one example, the soy protein concentrate with reduced phytic acid may comprise peptide (protein) of 40 to 95 % by weight, 45 to 95 % by weight, 50 to 90 % by weight, 55 to 80 % by weight, 50 to 70 % by weight, 50 to 60 % by weight, or 60 to 70 % by weight.

According to one example, the peptide content comprised in the soy protein concentrate with reduced phytic acid may be similar to the soy protein concentrate used as a raw material and the ratio of the content of the low molecular weight peptide (for example, peptide of less than 30kDa) to the total peptide may be higher than the soy protein concentrate used as a raw material. The content of the low molecular weight peptide (for example, peptide of less than 30kDa) comprised in the soy protein concentrate with reduced phytic acid according to one example may be increased than the raw material, and therefore, the soy protein concentrate with reduced phytic acid prepared by the method may have excellent digestibility.

According to one example, the soy protein concentrate with reduced phytic acid may comprise peptide of less than 30kDa of 10% to 95%, 15% to 90%, 15% to 85%, 15% to 80%, 20% to 90%, 20% to 80%, 30% to 90%, 30% to 80%, 30% to 70%, 30% to 65%, 30% to 60%, 30% to 55%, 30% to 50%, 30% to 45%, 30% to 40%, 20% to 40%, or 25% to 40% based on the total peptide comprised in the concentrate.

According to one example, the soy protein concentrate with reduced phytic acid may comprise peptide of less than 30kDa of 10 to 95 % by weight, 15 to 90 % by weight, 15 to 85 % by weight, 15 to 80 % by weight, 20 to 90 % by weight, 20 to 80 % by weight, 30 to 90 % by weight, 30 to 80 % by weight, 30 to 70 % by weight, 30 to 65 % by weight, 30 to 60 % by weight, 30 to 55 % by weight, 30 to 50 % by weight, 30 to 45 % by weight, 30 to 40 % by weight, 20 to 40 % by weight, or 25 to 40 % by weight based on the total peptide comprised in the concentrate.

The peptide means that amino acids of various combinations form a polymer by peptide bonds. The low molecular weight peptide is a peptide with a small molecular weight, and it has an advantage of being able to increase the digestibility when applied as animal feed by facilitating absorption during digestion. The higher content of low molecular weight peptide in the feed, the better the digestibility of animals. In one example, the low molecular weight peptide may refer to a peptide having a molecular weight of less than 40kDa, less than 30kDa or less than 25kDa.

The soy protein concentrate with reduced phytic acid, prepared using the composition for preparing a soy protein concentrate according to one example may have an increased content ratio of the peptide of less than 30kDa among the peptides comprised therein, and therefore, using the composition for preparing a soy protein concentrate according to one example or by the method for preparation according to one example, a soy protein concentrate with improved digestibility and a feed composition comprising the same may be prepared. According to one example, GPC (Gel Permeation Chromatography) may be used to measure the protein molecular weight distribution of the soy protein concentrate with reduced phytic acid.

According to one example, the soy protein concentrate with reduced phytic acid may be for use in animal feed preparation.

The animal may be a mammal, poultry, fish and/or crustacean. The mammal may include pigs, cattle, horses, deer, goats, dogs, cats and/or rabbits, and the poultry may include chickens, ducks, geese and/or turkeys, and the fish and crustacean may include trout, salmon, and/or shrimp.

In one example, the soy protein concentrate with reduced phytic acid may have increased digestibility (for example, phosphorus digestibility) than the soybean protein concentrate used as a raw material.

In the present description, "digestibility" may mean a fraction of nutriment (nutrients) that disappear from a gastrointestinal tract or a specific segment of a gastrointestinal tract, for example, the small intestine, and it may be used interchangeably with "nutrient digestibility" and/or "nutriment digestibility". The nutriment digestibility may be measured by a difference between what is administered to a subject and what is excreted in the subject's feces, or a difference between what is administered to a subject and what remains in a specific segment of a gastrointestinal tract, for example, a digest of ileum. In one example, the nutriment digestibility may be measured by a difference between nutrient intake by the total collection of excreta (feces) over a period of time and excreted nutriment; or may be measured by calculating the amount of nutriment lost in the entire gastrointestinal tract or a segment of the gastrointestinal tract, using an inactive marker (or indicator) that is not absorbed by an animal. This inactive marker (or indicator) may be titanium dioxide, chrome oxide (chromium oxide) and/or acid-insoluble ash. In one example, the digestibility may be expressed as a percentage of nutriment in feed or a mass unit of digestible nutriment per mass unit of nutriment in feed. In one example, the nutriment digestibility may include starch digestibility, fat digestibility, protein digestibility, mineral digestibility, phosphorus digestibility, and/or amino acid digestibility.

In one example, the nutrient digestibility may be calculated by Equation 1 below, and the nutrient below may be starch, carbohydrate, fat, protein, mineral and/or phosphorus.

(Equation 1)

Nutrient digestibility = 100 x (1- (amount of indicator of feed / amount of

indicator of feces) x (nutrient content of feces / nutrient content of feed))

Other aspect may provide a soy protein concentrate with reduced phytic acid prepared by the method for preparation.

Other aspect may provide a soy protein concentrate with reduced phytic acid obtained by reacting the composition for preparing a soy protein concentrate with reduced phytic acid.

Other aspect may provide a soy protein concentrate with reduced phytic acid obtained by reacting and drying the composition for preparing a soy protein concentrate with reduced phytic acid.

The soy protein concentrate with reduced phytic acid is the same as described above. Reacting and/or drying the composition is as described above.

The soy protein concentrate with reduced phytic acid according to one example is a vegetable high-protein material and may be utilized as an alternative material of fish meal used as a main protein source. In case of the fish meal, the price fluctuates greatly depending on the amount of fish caught, which is the raw material, and it is difficult to maintain the quality uniformly depending on the condition of the fish, while the soy protein concentrate is made from beans, a vegetable raw material, so quality control is possible and it may be used at a stable price due to relatively low variability in supply and demand. The conventional soy protein concentrate contains a number of high molecular weight proteins with a molecular weight of 1000kDa consisting of subunits of proteins and various anti-nutritional factors (ANF) inhibiting digestion, and therefore, there were factors inhibiting the digestive ability of an animal in the soy protein concentrate, but the soy protein concentrate with reduced phytic acid according to one example may have a lower phytic acid content than a raw material, have a higher content of low-protein peptide, and have the increased digestibility.

Other aspect may provide a feed composition comprising the soy protein concentrate with reduced phytic acid. Other aspect may provide a feed composition comprising a soy protein concentrate with reduced phytic acid prepared by the method for preparation. The 'soy protein concentrate with reduced phytic acid' comprised in the feed composition is as described above.

Herein, "feed composition" refers to a substance that maintains the life of an individual and supplies organic or inorganic nutrients necessary for rearing the individual. The feed composition may include nutrients such as energy, protein, lipid, vitamins and minerals, and the like, required by an individual who consumes feed, and is not particularly limited thereto, but may be vegetable feed such as grains, root fruits, food processing by-products, algae, fiber, oils and fats, starches, gourds, grain by-products, and the like, or animal feed such as proteins, inorganic materials, oils and fats, minerals, single cell proteins, animal planktons, fish meal, and the like. Herein, the feed composition is a concept including all materials added to feed (i.e., feed additive), feed raw materials or feed itself fed to an individual.

The individual refers to a breeding target and may be included without limitation as long as it is an organism capable of ingesting the feed of the present application. The feed composition according to one example may be applied to a number of animal diets, i.e., feed, including mammals, poultry, fish and/or crustaceans. The mammals include pigs, cattle, horses, deer, goats, dogs, cats, and/or rabbits, and the poultry includes chickens, ducks, geese, and/or turkeys, and the fish and crustaceans include trout, salmon, and/or shrimp.

According to one example, the feed composition may mix and use one or more kinds among organic acids such as citric acid, fumaric acid, adipic acid, and lactic acid; phosphate such as potassium phosphate, sodium phosphate, polymerized phosphate; and natural anti-oxidants such as polyphenol, catechin, tocopherol, vitamin C, green tea extract, keto acid, and tannic acid, in addition to the soy protein concentrate with reduced phytic acid for administration, and if necessary, other conventional additives such as anti-influenza agents, buffers, and/or bacteriostatic agents may be added. In addition, by additionally adding diluents, dispersants, surfactants, binders and/or lubricants, it may be formulated into injectable formulations such as an aqueous solution, suspension, emulsion, and the like, capsules, granules or tablets.

According to one example, the feed composition may be used with various kinds of supplements such as amino acids, inorganic salts, vitamins, anti-oxidants, antifungal agents, antibacterial agents, and the like, and in addition to main components such as vegetable protein feed including pulverized or crushed wheat, barley and corn, animal protein such as blood meal, meat meal, fish meal, animal fat and vegetable fat, nutritional supplements, growth promoters, digestion and absorption promoters and/or disease prevention agents, as an auxiliary component.

When the feed composition according to one example is used as a feed additive, the feed composition may be added as it is or used together with other components and may be appropriately used according to a common method. The administration form of the feed composition may be prepared as an immediate release or sustained release formulation in combination with a non-toxic pharmaceutically acceptable carrier. Such edible carrier may be corn starch, lactose, sucrose, and propylene glycol. The solid carrier may be in an administration form of tablets, powder, troche, and the like, and the liquid carrier may be in an administration form of syrup, liquid suspension, emulsion, solution and the like. In addition, the administration agent may contain a preservative, a lubricant, a solution accelerator, and a stabilizer.

The feed composition according to one example may comprise the soy protein concentrate with reduced phytic acid in an amount of about 1 to 500 g, or 100 to 500 g per 1 kg based on the dry weight.

In one example, the feed composition may further comprise one or more kinds selected from the group consisting of fish meal, wheat flour, soybean meal, soybean lecithin, fish oil, vitamins and minerals, in addition to the soy protein concentrate with reduced phytic acid. For example, the feed composition according to one example may comprise the soy protein concentrate with reduced phytic acid of 10 to 50 parts by weight, fish meal of 10 to 40 parts by weight, wheat flour of 10 to 40 parts by weight, soybean meal of 10 to 30 parts by weight, soybean lecithin of 0.1 to 10 parts by weight, fish oil of 0.1 to 10 parts by weight, vitamins of 0.01 to 1 part by weight, and minerals of 0.01 to 1 part by weight.

In one example, the vitamin may be ascorbic acid, DL-calcium pantothenate, choline bitrate, inositol, menadion, niacin, pyridoxine HCl, riboflavin, vitamin B1 (thiamine mononitrate), DL-alpha-tocopherol acetate (dl-a-Tocopherol acetate), retinyl acetate, biotin, folic acid, and/or cobalamin. In one example, the mineral may be NaCl, $MgSO_4 7H_2O$, $ZnSO_4 7H_2O$, Fe-Citrate, $MnSO_4$, $FeSO_4$, $CuSO_4$, calciumiodate, MgO, and/or $NaSeO_3$.

According to one example, the soy protein concentrate with reduced phytic acid may be mixed with additional feed raw materials and auxiliary ingredients and then supplied as a mash, or may be pelletized, expanded and/or extruded through an additional processing process.

The feed composition according to one example may have excellent digestibility equal or more than the soy protein concentrate and/or fish mean which are raw materials. For example, the feed composition may have digestibility (for example, phosphorus digestibility) of 60% or more, 65% or more, 70% or more, 75% or more, 80% or more, 85% or more, 60% to 99.9%, 60% to 99%, 60% to 95%, 60% to 90%, 60% to 85%, 65% to 99.9%, 65% to 99%, 65% to 95%, 65% to 90%, 65% to 85%, 70% to 99.9%, 70% to 99%, 70% to 95%, 70% to 90%, 70% to 85%, 75% to 99.9%, 75% to 99%, 75% to 95%, 75% to 90%, 75% to 85%, 80% to 99.9%, 80% to 99%, 80% to 95%, 80% to 90%, or 80% to 85%. The phosphorus digestibility may be calculated by Equation 1 or Equation 3.

Other aspect may provide a feed composition (for example, feed additive) for promoting (or increasing) digestibility (for example, phosphorus digestibility), comprising the soy protein concentrate with reduced phytic acid.

Other aspect may provide a feed composition (for example, feed additive) for replacing fish meal, comprising the soy protein concentrate with reduced phytic acid.

Other aspect may provide a method for increasing digestibility (for example, phosphorus digestibility), comprising administering the feed additive and/or feed composition according to one example into an animal.

[ADVANTAGEOUS EFFECTS]

Using the composition for preparing a soy protein concentrate with reduced phytic acid according to one example or by the method for preparation according to one example, the soy protein concentrate with reduced phytic acid can be efficiently prepared, and therefore, the content of phosphorus which can be used by animals can be increased, and the content ratio of small molecule peptide is increased to improve the digestibility of animals for the soy protein concentrate, and a feed composition comprising the soy protein concentrate with reduced phytic acid according to one example may have excellent digestibility.

[MODE FOR INVENTION]

Hereinafter, the present invention will be described in more detail by examples. However, these examples are intended to illustratively describe the present invention, but the scope of the present invention is not limited to these reference examples and examples.

Example 1. Method for preparation of soy protein concentrate with reduced phytic acid

A soy protein concentrate raw material was X-soy600 product supplied by CJ Selecta's affiliates.

After drying the soy protein concentrate raw material in a 105°C dry oven overnight, the weight before/after drying was measured to measure the moisture content of the corresponding raw material. As the pH of the raw material, the raw material and water (90% by weight) were mixed and 10% by weight solution was made and the pH thereof was measured. The soy protein concentrate raw material comprised about 60% by weight of protein, and the moisture content was about 7 to 8 % by weight, and the pH was 6.5 to 6.8.

After confirming the moisture content and pH of the raw material, the amount of added water and the amount of hydrochloric acid to be added according to each enzyme reaction condition were calculated. The final moisture content was calculated by Equation 2 below, and the amount of hydrochloric acid to be added in Equation 2 below was calculated according to the desired pH.

(Equation 2)

Final moisture content %(% by weight) = (raw material weight (g) x moisture

content % of raw material (% by weight) + amount of added water to be added (g)

+ amount of hydrochloric acid to be added (g))/(raw material weight (g) + amount

of added water to be added (g) + amount of hydrochloric acid to be added (g)) x 100

By hydrochloric acid and/or water to the soy protein concentrate raw material, the moisture content of the composition comprising the soy protein concentrate was adjusted to be 40 to 60 % by weight based on the total weight of the composition and have pH 4.5 to 6.8. For pH adjustment of the composition comprising the soy protein concentrate, hydrochloric acid was used, and only water was added to the raw material pH 6.8 reactant. In the present example, the pH of the reaction raw material was adjusted using 1N HCl. 1N HCl 40ml to adjust the pH of the soy protein concentrate raw material of 100g to 4.5, 1N HCl 33.5ml to adjust the pH to 4.75, 28ml to adjust the pH to 5, and 1N HCl 15ml to adjust the pH to 5.5 were added. Phytase (Bision biochem) was added by mixing with water to be added to adjust the moisture content of the composition comprising the soy protein concentrate (0.005 to 0.05 % by weight based on the soy protein concentrate raw material), and this was reacted at a reaction temperature of 60°C for 2 hours to 8 hours to prepare a soy protein concentrate with reduced phytic acid.

In addition to compare the effect according to the phytase concentration to be added, by reacting the composition comprising a soy protein concentrate, which comprised moisture of 60 % by weight (based on the total composition) and phytase at various concentrations (0.005 % by weight to 0.5 % by weight based on the weight of the soy protein concentrate raw material) and had pH 4.5, soy protein concentrates with reduced phytic acid in various contents were prepared. After completing the reaction, all samples were dried by freeze drying and pulverized, to prepare a soy protein concentrate with reduced phytic acid in a powder form.

Example 2. Measurement of phytic acid content and free phosphorus content of soy protein concentrate with reduced phytic acid

As the method of Example 1, for the prepared soy protein concentrate with reduced phytic acid, the phytic acid content was measured using a phytic acid assay kit (Megazyme) according to the manual of the manufacturer.

In order to examine the phytic acid content of the soy protein concentrate according to the phytase content and reaction time, the phytic acid content of the soy protein concentrate with reduced phytic acid (% by weight; based on the dried matter weight of the soy protein concentrate with reduced phytic acid), prepared by reacting the composition comprising a soy protein concentrate, which comprises moisture of 60 % by weight (based on the total composition), and comprises phytase in a range of 0.005 % by weight to 0.5 % by weight (based on the raw material weight), and have pH 4.5, for 2 to 4 hours, was described in Table 1 below.

In Table 1, 'raw material' refers to a soy protein concentrate raw material used to prepare a soy protein concentrate with reduced phytic acid, and the moisture content of the raw material was about 7 to 8 % by weight, and the pH was 6.5 to 6.8, and the phytase was not comprised. In Table 1 below, 0.005% to 0.5% comprised in the composition means the content of phytase (% by weight based on the weight of the soy protein concentrate raw material).

[Table 1]

| Time (hr) | 0.005% | 0.010% | 0.025% | 0.050% | 0.075% | 0.100% | 0.250% | 0.500% |
|---|---|---|---|---|---|---|---|---|
| Raw material | 2.03 | 2.03 | 2.03 | 2.03 | 2.10 | 1.99 | 1.99 | 2.04 |
| 2 hours | 1.24 | 1.02 | 0.78 | 0.59 | 0.60 | 0.60 | 0.58 | - |
| 4 hours | 1.08 | 0.82 | 0.61 | 0.56 | 0.53 | 0.49 | 0.35 | 0.40 |

(continued)

| | | | | | | | | |
|---|---|---|---|---|---|---|---|---|
| 8 hours | 0.87 | 0.78 | 0.57 | 0.46 | 0.45 | 0.31 | 0.33 | 0.31 |

As shown in Table 1, the composition prepared in Example 1 had a significantly reduced phytic acid content based on the raw material.

In addition, for the soy protein concentrate with reduced phytic acid prepared by the method of Example 1 (prepared by reacting the composition comprising a soy protein concentrate, which comprises phytase of 0.05 % by weight based on the weight of the soy protein concentrate raw material, and comprises moisture of 60 % by weight based on the total composition and has the pH 4.5 for 4 hours) and a soy protein concentrate with not reduced phytic acid (control group), the free phosphorus content (% by weight; based on the dried matter weight of the soy protein concentrate with reduced phytic acid) and $PO_4^-$ content (% by weight; based on the dried matter weight of the soy protein concentrate with reduced phytic acid) were measured and described in Table 2.

Free phosphorus was calculated based on the absorbance value derived through a megazyme kit assay process, and this refers to the phosphorus content isolated in the original sample without enzymatic action such as phytase or phosphatase in the phytic acid extraction solution in the kit assay process. The absorbance was measured at 655nm, and the free phosphorus absorbance in the kit was inserted to the absorbance value of the total phosphorus, and the absorbance value of free phosphorus was calculated as 0, and thereby, the phosphorus content was calculated. In other words, the phosphorus content calculated from the free phosphorus absorbance was confirmed to derive the content of the free phosphorus itself.

$PO_4^-$ is an anionic form of phosphorus dissolved in a specific solvent, and the result value may be different depending on the extraction solvent and method, but the $PO_4^-$ content was measured using two methods such as normal temperature extraction using water as a solvent and hot water extraction through a heating process. The normal temperature extract was extracted at a room temperature for 20 minutes by diluting a sample (composition comprising a soy protein concentrate with reduced phytic acid) and water to a certain number of times, and the hot water extract was additionally extracted in boiling water for 20 minutes after normal temperature extraction. After centrifugation of the corresponding extract, the $PO_4^-$ content was measured using the supernatant as a sample by ion chromatography.

[Table 2]

| Sample | Extraction method | Phytic acid content (% by weight, based on the dried matter) | Phosphorus content (% by weight, based on the dried matter) | |
|---|---|---|---|---|
| | | | Free phosphorus | $PO_4^-$ |
| Soy protein concentrate (Control group) | Water extraction | 1.92 | 0.09 | 0.20 |
| | Hot water extraction | | | 0.25 |
| Soy protein concentrate with reduced phytic acid | Water extraction | 0.54 | 0.49 | 1.64 |
| | Hot water extraction | | | 1.53 |

As shown in Table 2, it could be confirmed that the soy protein concentrate (control group) comprised free phosphorus of 0.08~0.09 % by weight (based on the dried matter of the soy protein concentrate with reduced phytic acid), and the $PO_4^-$ content was 0.2 % by weight (based on the dried matter of the soy protein concentrate with reduced phytic acid) in all the samples collected in two extraction methods of normal temperature extraction and hot water extraction. Free phosphorus means the phosphorus content itself, and the $PO_4^-$ content measured by ion chromatography reflected not only phosphorus but also oxygen (O) molecular contents. As shown in Table 2, free phosphorus comprised in the soy protein concentrate with reduced phytic acid was about 0.5 % by weight, and the phosphorus content compared to the control group was increased about 5~6 times. In addition, the $PO_4^-$ content of the soy protein concentrate with reduced phytic acid was 1.53 to 1.64 % by weight, although there was a slight difference depending on the extraction method, and the $PO_4^-$ content was increased 7~8 times compared to the control group.

Accordingly, it could be confirmed that the soy protein concentrate with reduced phytic acid, prepared by the method of Example 1 had the significantly increased content of phosphorus in a form capable of being easily used in the process of digestion and absorption of feed by animals.

Phosphate is an additive used to supplement the content of phosphorus in a mixed feed, and mono-, di-, tricalcium phosphate, and the like are widely used. The soy protein concentrate with reduced phytic acid may increase the phosphorus content in this phosphate form and enhance the nutritional value of other components in addition to a protein source, and give an economic effect that can reduce the use of an additive of a phosphorus source in the whole mixed feed.

In addition, the effect according to the moisture content and pH of the composition for preparing a soy protein concentrate with reduced phytic acid was to be examined. Accordingly, similarly to the method of Example 1, the composition, which comprises a soy protein concentrate raw material, comprises moisture of 40 to 60 % by weight based on the total composition, comprises phytase of 0.05 % by weight based on the raw material of the soy protein concentrate, has pH of 4.5 to 6.8, and comprises a soy protein concentrate, was reacted at 60°C for 2 to 8 hours, dried and pulverized, to prepare a soy protein concentrate with reduced phytic acid in a powder form, and the content of free phosphorus for that (% by weight; based on the dried matter weight of the soy protein concentrate with reduced phytic acid) was measured by the above method and shown in Table 3, and the phytic acid content (% by weight: based on the dried matter weight of the soy protein concentrate with reduced phytic acid) was measured by the above method and shown in Table 4.

[Table 3]

| Moisture content (% by weight) | Reaction time (hr) | pH | | | | |
|---|---|---|---|---|---|---|
| | | 4.5 | 4.75 | 5 | 5.5 | 6.8 |
| Raw material | - | 0.09 | | | | |
| 40% | 2 | 0.33 | - | - | - | - |
| | 4 | 0.40 | - | - | - | - |
| | 8 | 0.46 | - | - | - | - |
| 50% | 2 | 0.44 | - | - | - | - |
| | 4 | 0.48 | - | - | - | - |
| | 8 | 0.53 | - | - | - | - |
| 55% | 2 | 0.47 | 0.45 | 0.44 | - | - |
| | 4 | 0.51 | 0.50 | 0.48 | - | - |
| | 8 | 0.54 | 0.53 | 0.52 | - | - |
| 60% | 2 | 0.54 | 0.50 | 0.49 | 0.40 | 0.21 |
| | 4 | 0.56 | 0.54 | 0.53 | 0.43 | 0.29 |
| | 8 | 0.58 | 0.56 | 0.55 | 0.48 | 0.32 |

As shown in Table 3, it could be confirmed that the composition for preparing a soy protein concentrate with reduced phytic acid according to one example may increase the free phosphorus content, and the free phosphorus content in the soy protein concentrate with reduced phytic acid according to one example may be significantly increased.

[Table 4]

| Moisture content (% by weight) | Reaction time (hr) | pH | | | | |
|---|---|---|---|---|---|---|
| | | 4.5 | 4.75 | 5 | 5.5 | 6.8 |
| Raw material | - | 1.98 | | | | |
| 40% | 2 | 1.20 | - | - | - | - |
| | 4 | 1.03 | - | - | - | - |
| | 8 | 0.89 | - | - | - | - |

(continued)

| Moisture content (% by weight) | Reaction time (hr) | pH | | | | |
|---|---|---|---|---|---|---|
| | | 4.5 | 4.75 | 5 | 5.5 | 6.8 |
| 50% | 2 | 0.87 | - | - | - | - |
| | 4 | 0.73 | - | - | - | - |
| | 8 | 0.53 | - | - | - | - |
| 55% | 2 | 0.66 | 0.75 | 0.71 | - | - |
| | 4 | 0.54 | 0.59 | 0.64 | - | - |
| | 8 | 0.37 | 0.49 | 0.52 | - | - |
| 60% | 2 | 0.62 | 0.81 | 0.57 | 1.03 | 1.46 |
| | 4 | 0.58 | 0.62 | 0.60 | 0.86 | 1.33 |
| | 8 | 0.51 | 0.48 | 0.58 | 0.75 | 1.26 |

As shown in Table 4, it could be confirmed that the composition for preparing a soy protein concentrate with reduced phytic acid according to one example could reduce the phytic acid content, and in the soy protein concentrate with reduced phytic acid according to one example, the phytic acid content was significantly reduced.

Example 3. Analysis pf characteristics of soy protein concentrate with reduced phytic acid

For the soy protein concentrate with reduced phytic acid prepared by the method of Example 1 (prepared by reacting the composition comprising phytase of 0.05 % by weight based on the soy protein concentrate raw material, comprising moisture of 60 % by weight based on the total composition, having pH 4.5 to 6.8 and comprising a soy protein concentrate for 4 hours and then drying), the protein degradability (Gel Permeation Chromatography; GPC), protein content (% by weight: based on the dried matter weight of the soy protein concentrate with reduced phytic acid) and the phytic acid content (% by weight: based on the dried matter weight of the soy protein concentrate with reduced phytic acid) were measured and they were shown in Table 5.
The protein content (% by weight) comprised in the soy protein concentrate with reduced phytic acid was measured by Kjeldahl instrumental analysis.
For GPC, about 100mg of a sample (soy protein concentrate with reduced phytic acid) was mixed with 5ml of 8M urea solution, sonicated and centrifuged (8000rpm, 10 minutes), and then the recovered supernatant was used as a sample and analyzed with a GPC column. When deriving the analysis result, standard proteins with different molecular weights were analyzed to confirm the retention time (RT) of elution and a standard curve was calculated from the correlation between the molecular weight and RT, and accordingly, the distribution of the protein molecular weight in the sample to be measured was able to derived. Specifically, the RT at which a protein of less than 30kDa was detected was measured, and the ratio of the protein of less than 30kDa comprised in the soy protein concentrate with reduced phytic acid was calculated as the ratio of the area in the RT section representing less than 30kDa among the total chromatogram area and described in Table 5. Then, the total area was calculated after excluding from the total chromatogram from the RT section in which the peak of the 8M urea extraction solvent appeared among the result.
As shown in Table 5, the soy protein concentrate (control group, raw material) comprised phytic acid of about 2 % by weight (based on the dried matter weight), and as examined in Example 2, the phytic acid content of the soy protein concentrate with reduced phytic acid prepared by the method according to one example was reduced.
In addition, as the result of GPC analysis, the protein content and peptide content of less than 30kDa comprised in the soy protein concentrate with reduced phytic acid were similar to or increased than the soy protein concentrate used as a raw material.

[Table 5]

| | Raw material | 4.5 | 4.75 | 5 | 5.5 | 6.8 |
|---|---|---|---|---|---|---|
| Phytic acid (% by weight, based on the dried matter) | 2.02 | 0.58 | 0.62 | 0.60 | 0.86 | 1.33 |
| Protein (% by weight, based on the dried matter) | 64.08 | 63.35 | 63.53 | 63.51 | 63.78 | 63.94 |
| GPC : <30kDa ratio | 26.92 | 38.85 | 37.96 | 35.83 | 32.09 | 26.02 |

Example 4. Specification evaluation of feed comprising soy protein concentrate with reduced phytic acid

In the present example, the digestibility of the soy protein concentrate with reduced phytic acid prepared by the method of Example 1 (prepared by reacting the composition comprising phytase of 0.05 % by weight based on the soy protein concentrate raw material, comprising moisture of 60 % by weight based on the total composition, having pH 4.5 to 6.8 and comprising a soy protein concentrate and then drying) and fish meal not containing phytic acid was measured.

Specifically, Litopenaues vannamei (purchased from Palddack Shrimp located in Goseong, Jeollanam-do) was pre-bred by supplying basic feed for 2 weeks, and a breeding experiment was conducted for 4 weeks. Pre-bred Litopenaues vannamei (average fish weight 14.6±0.34g) was randomly placed in 3 repetitions of 10 each, and the experimental feed comprising the composition described in Table 6 below was supplied 4 times a day at 6% of the fish weight, respectively, and feces was collected during breeding. During the experiment period, each water tank was maintained at 29 ± 1°C and the salinity of the breeding water was maintained in the range of 33 ± 1 ppt. The oxygen supply amount was 6ppm, and the flow rate was maintained the same at 0.8 L/min. The experimental feed for a breeding experiment was supplied by 6% of the fish weight 4 times a day. Fish meal (Anchovy fish meal, Peru), wheat flour, soybean meal, soybean lecithin, fish oil, Vitamin Mix and Mineral Mix were used for feed preparation. The composition of the used vitamin mix is as follows: (vitamin mix (mg/kg in diets) : Ascorbic acid, 300 mg/kg; dl-Calcium pantothenate, 150 mg/kg; Choline bitrate, 3000 mg/kg; Inositol, 150 mg/kg; Menadion, 6 mg/kg; Niacin, 150 mg/kg; Pyridoxine HCl, 15 mg/kg; Riboflavin, 30 mg/kg; Thiamine mononitrate, 15 mg/kg; dl-a-Tocopherol acetate, 201 mg/kg; Retinyl acetate, 6 mg/kg; Biotin, 1.5 mg/kg; Folic acid, 5.4 mg/kg; Cobalamin, 0.06). The composition of the used mineral mix is as follows: (mineral mix (mg/kg in diets) : NaCl, 437.4 mg/kg; $MgSO_4 \cdot 7H_2O$, 1379.8 mg/kg; $ZnSO_4 \cdot 7H_2O$, 226.4 mg/kg; Fe-Citrate, 299 mg/kg; $MnSO_4$, 0.016 mg/kg; $FeSO_4$, 0.0378 mg/kg; $CuSO_4$, 0.00033 mg/kg; Calciumiodate, 0.0006; MgO, 0.00135 mg/kg; $NaSeO_3$, 0.00025.)

[Table 6]

| Composition | FM (% by weight, based on the dried matter) | DPS (% by weight, based on the dried matter) |
|---|---|---|
| Fish meal (Anchovy fish meal, Peru) | 24.50 | 24.50 |
| Wheat flour | 21.86 | 21.86 |
| Soybean meal | 19.88 | 19.88 |
| Soybean lecithin | 1.68 | 1.68 |
| Fish oil | 1.40 | 1.40 |
| Vitamin Mix | 0.18 | 0.18 |
| Mineral Mix | 0.18 | 0.18 |
| Fish meal (Anchovy fish meal, Peru) | 30 | - |
| Soy protein concentrate with reduced phytic acid | - | 30 |
| Chromic oxide | 0.35 | 0.35 |
| Total | 100 | 100 |

The feed digestibility was measured by measuring the content of chromium oxide used as an indicator in the experimental feed supplied to Litopenaues vannamei and feces. To oxidize chromium oxide (indicator) to a monochromate form, 5-10mg of a sample (experimental feed or feces) was weighted and transferred to a glass test tube, and 4ml of perchloric reagent was added, and this was heated at 300 °C for 20 minutes, and transferred to a glass flask, and tertiary distilled water was added to adjust the volume of the sample to 25ml, and the absorbance at 350nm was measured. The phosphorus digestibility (%) of the feed was calculated by calculating the content of phosphorus as a nutrient using Equation 3 below, and the result was shown in Table 7 below.

(Equation 3)

Apparent Digestible Coefficient (ADC)

= 100 x (1- (amount of indicator of feed / amount of indicator of feces) x

(nutrient content of feces / nutrient content of feed))

As shown in Table 7, in case of the feed comprising the soy protein concentrate with reduced phytic acid according to one example (DPS), the phosphorus digestibility was 80.8%, and in case of the feed comprising fish meal instead of the soy protein concentrate with reduced phytic acid (FM), the phosphorus digestibility was 81.4%, and there was no statistically significant difference ($p < 0.05$) between two feeds, and thereby, it could be confirmed that the phosphorus digestibility was at an equal level. From these results, it could be found that the soy protein concentrate with reduced phytic acid according to one example might be used as protein feed capable of replacing fish meal.

[Table 7]

|  | FM | DPS |
| --- | --- | --- |
| Phosphorus digestibility (%,ADC) | 81.4 | 80.8 |

Claims

1. A composition for preparing a soy protein concentrate with reduced phytic acid,

    comprising a soy protein concentrate and phytase,
    wherein the content of the phytase is 0.005 to 0.5 % by weight based on the weight of the soy protein concentrate, and
    the moisture content is 40 % by weight or more based on the weight of the total composition, and
    the pH is 6.8 or less.

2. The composition for preparing a soy protein concentrate with reduced phytic acid according to claim 1, wherein the content of the phytase is 0.025 to 0.5 % by weight based on the weight of the soy protein concentrate.

3. The composition for preparing a soy protein concentrate with reduced phytic acid according to claim 1, wherein the moisture content is 50 % by weight or more based on the weight of the total composition.

4. The composition for preparing a soy protein concentrate with reduced phytic acid according to claim 1, wherein the pH is 5.5 or less.

5. A method for preparation of a soy protein concentrate with reduced phytic acid, comprising reacting the composition according to any one of claims 1 to 4.

6. The method for preparation of a soy protein with reduced phytic acid according to claim 5, further comprising drying the reactant reacted in the above step.

7. The method according to claim 5, wherein the soy protein concentrate with reduced phytic acid comprises phytic acid of less than 1.5 % by weight.

8. The method according to claim 5, wherein the soy protein concentrate with reduced phytic acid comprises free phosphorus of 0.2 to 1 % by weight.

9. The method according to claim 5, wherein the soy protein concentrate with reduced phytic acid comprises peptides of less than 30kDa in an amount of 15% to 90% based on the total peptides.

10. The method according to claim 5, wherein the soy protein concentrate with reduced phytic acid is for using in animal feed preparations.

11. The method according to claim 5, wherein the soy protein concentrate with reduced phytic acid has increased phosphorus digestibility than the soy protein concentrate used as a raw material.

12. A soy protein concentrate with reduced phytic acid,

> comprising the soy protein concentrate with reduced phytic acid obtained by reacting the composition according to any one of claims 1 to 4, and
> comprising phytic acid of less than 1.5 % by weight.

13. The soy protein concentrate with reduced phytic acid according to claim 12, comprising free phosphorus of 0.2 to 1 % by weight.

14. The soy protein concentrate with reduced phytic acid according to claim 12, comprising peptides of less than 30kDa in an amount of 15% to 90% based on the total peptides.

15. A feed composition comprising the soy protein concentrate with reduced phytic acid according to claim 12.

16. The feed composition according to claim 15, further comprising one or more kinds selected from the group consisting of fish meal, wheat flour, soybean meal, soybean lecithin, fish oil, vitamins and minerals.

## INTERNATIONAL SEARCH REPORT

| International application No. |
| --- |
| **PCT/KR2020/015092** |

**A.     CLASSIFICATION OF SUBJECT MATTER**

**A23J 3/16**(2006.01)i; **C12N 9/16**(2006.01)i

According to International Patent Classification (IPC) or to both national classification and IPC

**B.     FIELDS SEARCHED**

Minimum documentation searched (classification system followed by classification symbols)

A23J 3/16(2006.01); A01H 5/00(2006.01); A23J 3/34(2006.01); C12N 1/00(2006.01); C12N 9/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 피타제(phytase), 피틴산(phytic acid), 대두(soybean), 단백질(protein), 인 (phosphorus)

**C.     DOCUMENTS CONSIDERED TO BE RELEVANT**

| Category* | Citation of document, with indication, where appropriate, of the relevant passages | Relevant to claim No. |
| --- | --- | --- |
| X | KR 10-2003-0038783 A (FUJI OIL CO., LTD.) 16 May 2003 (2003-05-16)<br>    See example 10; and claim 7. | 1-7,12 |
| Y | | 8-11,13-16 |
| Y | JP 2002-087979 A (FUJI OIL CO., LTD.) 27 March 2002 (2002-03-27)<br>    See paragraph [0026]; example 1; and claims 1 and 8. | 8,13 |
| Y | CN 102396643 A (SOUTH CHINA UNIVERSITY OF TECHNOLOGY) 04 April 2012 (2012-04-04)<br>    See abstract; and claim 1. | 9,14 |
| Y | JP 06-319539 A (AVEVE NV) 22 November 1994 (1994-11-22)<br>    See abstract; example 12; and claims 1, 13, 15, 18, 19 and 21. | 10-11,15-16 |
| A | KR 10-2001-0081175 A (KIM, Hyeung Rak) 29 August 2001 (2001-08-29)<br>    See entire document. | 1-16 |

| ☐ Further documents are listed in the continuation of Box C. | ☑ See patent family annex. |
| --- | --- |

| *    Special categories of cited documents:<br>"A"  document defining the general state of the art which is not considered<br>       to be of particular relevance<br>"D"  document cited by the applicant in the international application<br>"E"  earlier application or patent but published on or after the international<br>       filing date<br>"L"  document which may throw doubts on priority claim(s) or which is<br>       cited to establish the publication date of another citation or other<br>       special reason (as specified)<br>"O"  document referring to an oral disclosure, use, exhibition or other<br>       means<br>"P"  document published prior to the international filing date but later than<br>       the priority date claimed | "T"  later document published after the international filing date or priority<br>       date and not in conflict with the application but cited to understand the<br>       principle or theory underlying the invention<br>"X"  document of particular relevance; the claimed invention cannot be<br>       considered novel or cannot be considered to involve an inventive step<br>       when the document is taken alone<br>"Y"  document of particular relevance; the claimed invention cannot be<br>       considered to involve an inventive step when the document is<br>       combined with one or more other such documents, such combination<br>       being obvious to a person skilled in the art<br>"&"  document member of the same patent family |
| --- | --- |

| Date of the actual completion of the international search | Date of mailing of the international search report |
| --- | --- |
| **17 February 2021** | **17 February 2021** |

| Name and mailing address of the ISA/KR<br><br>**Korean Intellectual Property Office**<br>**Government Complex-Daejeon Building 4, 189 Cheongsa-**<br>**ro, Seo-gu, Daejeon 35208** | Authorized officer |
| --- | --- |
| Facsimile No. **+82-42-481-8578** | Telephone No. |

Form PCT/ISA/210 (second sheet) (July 2019)

<table>
<tr><td colspan="2" align="center"><strong>INTERNATIONAL SEARCH REPORT</strong><br>Information on patent family members</td><td colspan="2">International application No.<br><strong>PCT/KR2020/015092</strong></td></tr>
</table>

| Patent document cited in search report | | | Publication date (day/month/year) | Patent family member(s) | | | Publication date (day/month/year) |
|---|---|---|---|---|---|---|---|
| KR | 10-2003-0038783 | A | 16 May 2003 | AU | 2001-292291 | B2 | 02 March 2006 |
| | | | | AU | 9229101 | A1 | 15 April 2002 |
| | | | | CN | 1466420 | A | 07 January 2004 |
| | | | | CN | 1466420 | C | 25 January 2006 |
| | | | | EP | 1323353 | A1 | 02 July 2003 |
| | | | | EP | 1323353 | B1 | 01 April 2009 |
| | | | | JP | 4453253 | B2 | 21 April 2010 |
| | | | | KR | 10-0754051 | B1 | 31 August 2007 |
| | | | | US | 2004-0028799 | A1 | 12 February 2004 |
| | | | | US | 7226633 | B2 | 05 June 2007 |
| | | | | WO | 02-28198 | A1 | 11 April 2002 |
| JP | 2002-087979 | A | 27 March 2002 | None | | | |
| CN | 102396643 | A | 04 April 2012 | None | | | |
| JP | 06-319539 | A | 22 November 1994 | AT | 244302 | T | 15 July 2003 |
| | | | | CA | 2120265 | A1 | 06 October 1994 |
| | | | | CA | 2120265 | C | 29 May 2007 |
| | | | | DE | 69333071 | T2 | 06 May 2004 |
| | | | | DK | 0619369 | T3 | 06 October 2003 |
| | | | | EP | 0619369 | A1 | 12 October 1994 |
| | | | | EP | 0619369 | B1 | 02 July 2003 |
| | | | | EP | 0619369 | B2 | 30 September 2009 |
| | | | | ES | 2202305 | T3 | 01 April 2004 |
| | | | | FI | 941545 | A | 06 October 1994 |
| | | | | JP | 3587876 | B2 | 10 November 2004 |
| | | | | NO | 324857 | B1 | 17 December 2007 |
| | | | | NO | 941183 | L | 06 October 1994 |
| | | | | PT | 619369 | E | 28 November 2003 |
| | | | | US | 5443979 | A | 22 August 1995 |
| | | | | US | 5554399 | A | 10 September 1996 |
| KR | 10-2001-0081175 | A | 29 August 2001 | None | | | |

Form PCT/ISA/210 (patent family annex) (July 2019)

EP 4 052 587 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100279367 A